# EUROPEAN PATENT APPLICATION

(11) **EP 3 862 414 A1**
(43) Date of publication of application: **11.08.2021**
(21) Application number: 21020048.1
(22) Date of filing: 03.02.2021
(51) Int. Cl.: C12M 1/04, C12M 1/00

(54) **BIOREACTOR**

(30) Priority: 07.02.2020 IT 202000002338
(71) Applicant: Rigenerand S.r.l., 41036 Medolla (MO) (IT)
(72) Inventor: Candini, Olivia, 40014 Crevalcore (BO) (IT); Dominici, Massimo, 44100 Ferrara (FE) (IT); Mari, Giorgio, 41036 Mirandola (MO) (IT)
(74) Representative: GCA S.R.L.

(57) **Abstract**

The bioreactor (1) comprises a box-like body (4) having an internal culture chamber (5); a three-dimensional support (6) fitted in said internal chamber (5) for cells (2, 3) to catch on and grow and which divides said internal chamber (5) into a first culture half-chamber (7) and a second culture half-chamber (8); a first opening (9) obtained in the box-like body (4) in correspondence with said first half-chamber (7); a second opening (10) obtained in the box-like body (4) in correspondence with said second half-chamber (8); at least one outward vent opening (13) which is obtained in said body (4) in correspondence with said first half-chamber (7).

## Description

### Field of the invention

The invention concerns a bioreactor, generally usable for the culture and growth of cells.

### Background of the invention

Devices are known, used in the biological field for the in-vitro culture of cells and for the formation of organic tissues.

Typically, these devices consist of a container body inside which a culture chamber is defined in which a three-dimensional support is fitted, intended to receive and support the cells transported in suspension by a solution.

The container body has a loading opening for the transport solutions and an outlet for the exhausted solutions.

Gas-permeable surfaces are also provided in these devices, to provide adequate oxygenation of the cells and prevent apoptosis due to anoxia.

These surfaces, typically in the form of membranes, are also hydrophobic, to prevent the loss of solutions or the entry of contaminating liquids from the outside.

A device for cell culture is known from patent EP2321399-B1, property of the present Applicant, which comprises a container body which has an internal compartment and which contains a quantity of cells to be cultivated and a culture surface fitted in the internal compartment.

The container body is equipped with an entrance opening for the cells to be cultivated, and is formed by two facing half-shells which define the internal compartment between them.

The two shells are reciprocally attached by removable adhesive means and at least one of the shells is made of a gas-permeable material.

Patent US5843766 discloses an apparatus for the culture and packaging of three-dimensional cultivations of tissues.

The apparatus comprises a box-like housing that has a culture compartment inside it, in which a substrate is fitted on which to prepare the growth of the tissue.

In the internal compartment there is also a fluid distributor which has the function of spreading the cells for the formation of the tissue on the entire surface of the substrate.

The box-like housing is equipped with a loading opening for a fluid in which cells are transported, and a discharge opening to eliminate the exhausted fluid.

The entire box-like housing is made watertight to prevent the contact of the cultured cells with external pollutants.

This state of the art has some disadvantages.

One disadvantage is that it does not allow the simultaneous culture of cells of different types and nature in the same culture medium inside the same bioreactor, even if the culture takes place in separate culture half-chambers.

Furthermore, if for a certain type of cells it is necessary to use a specific culture medium in a gel state loaded in a half-chamber, the gel, due to its viscosity, after being loaded into the intended culture half-chamber, is not able to imbue the substrate that is in the culture compartment and irreversibly occludes the openings of the half-chamber in which it is loaded.

This, when a liquid transport solution of other cells of a different type is loaded into the other half-chamber, causes an increase in pressure inside the bioreactor and in both half-chambers, which opposes the loading of the cells transported with the liquid solution.

Considering the widespread use of semi-solid media, such as hydrogels or liquids with increased viscosity for the cultivation of specific cell types or for the growth of cells in spheroidal structures, as well as the need to reproduce different tissue layers to study their interaction, the culture of cells in different types of media inside the same bioreactor is able to generate complex co-culture models in the service of basic research and diagnostics.

### Purposes of the invention

One purpose of the invention is to improve the state of the art.

Another purpose of the invention is to manufacture a bioreactor that allows to simultaneously cultivate and grown cells inside it, in different culture media.

A further purpose of the invention is to manufacture a bioreactor that allows, if required, to predict interactions between cells grown in different media during their culture in the same bioreactor.

A further purpose of the invention is to expand the current research possibilities.

According to one aspect of the invention, a bioreactor is provided, in accordance with the characteristics of claim 1.

The invention allows to achieve the following advantages:
- simultaneously grow cells in different culture media inside the same bioreactor;
- allow interactions between cells in different types of media inside the same bioreactor;
- significantly expand research possibilities.

### Brief description of the drawings

Other characteristics and advantages of the invention will become more apparent from the description of some preferred, but not exclusive, embodiments of a bioreactor, given as a non-restrictive example in the attached drawings wherein:

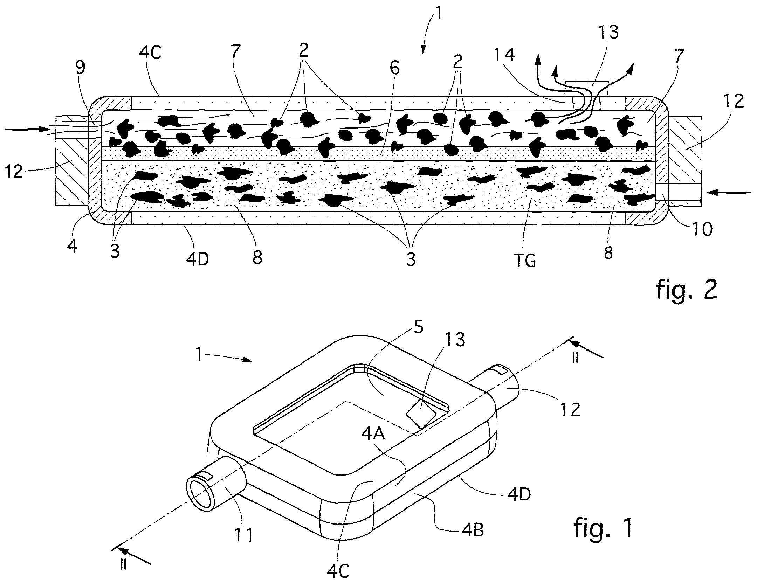

Fig. 1 is a schematic and perspective view of a bioreactor according to the invention;
Fig. 2 is a vertical section view, taken according to tracing plane II-II of fig 1.

### Detailed description of a preferred example embodiment

With reference to the drawings as above, 1 indicates a bioreactor for the culture of cells, in the specific case shown in the drawings, cells 2 and 3 grown in different culture media.

The bioreactor 1 is formed by a containing body 4 inside which there is defined an internal culture chamber 5 in which a three-dimensional support 6 is housed which, in practice, divides the chamber 5 into two half-chambers, indicated as first culture half-chamber 7 and second culture half-chamber 8.

The body 4 has a flattened shape and is preferably formed by two identical shells 4A and 4B coupled to each other, made integral with known joining means, such as for example a double-sided adhesive substance or an ultrasound welding.

The body 4 is equipped with at least two openings for connection to the outside, namely a first opening 9 and a second opening 10 in the drawings, both equipped with respective mouths 11 and 12 of a standardized type for possible connections with pipes and/or external apparatuses.

The three-dimensional support 6 is gas-permeable but water-repellant and consequently does not allow the passage of culture media between the two half-chambers 7 and 8.

According to the invention, in at least one of these two there is provided a vent opening 13 through which air is evacuated during the loading of a culture medium into the respective half-chamber, in the example shown in the drawings, into the half-chamber 7.

As can be seen from the drawings, the vent opening 13 is obtained in the first half-chamber 7 on the opposite side with respect to the first opening 9.

The vent opening 13 is equipped with gas-permeable and hydrophobic filtering means 14, capable of maintaining sterility inside the bioreactor 1.

The body 4, as mentioned, has a flattened shape and defines two larger surfaces 4C and 4D opposite and parallel to each other, at least one of which, in the preferred embodiment of the bioreactor 1, comprises a respective oxygenation membrane, hydrophobic and capable of sterilizing air, in order to supply oxygen to the two half-chambers 7 and 8.

The functioning of the bioreactor is as follows: a medium TG in the form of a gel is loaded, through the second opening 10, into the half-chamber 8 inside which the cells 3 are dispersed.

A culture medium TL in liquid form, basically a solution, which carries the cells 2 is loaded, through the first opening 9, into the first half-chamber 7.

During the loading of the liquid medium TL, the air that is present in the culture chamber 5 is automatically evacuated through the vent opening 13, passing through the filtering means 14 which, since they are hydrophobic but gas-permeable, protect the culture chamber 5 from external contaminations.

The evacuation of the air is favored by the position of the vent opening 13, obtained in the first half-chamber 7 on the opposite side with respect to the first opening 9.

In this way, the pressure inside the culture chamber 5 is kept constant and equal to the atmospheric pressure and the inflow of the liquid medium TL into the half-chamber 7 is not opposed.

The bioreactor 1 therefore allows the culture of cells 2 and 3 in different culture media (TL and TG), possibly interacting with each other through the three-dimensional support 6.

The cell culture method in the bioreactor 1 comprises: loading the second culture medium in a gel or semisolid state TG into the second culture half-chamber 8 and subsequently the first culture medium in the liquid state TL into the first half-chamber 7.

In order to allow the correct and easy loading of the liquid culture medium TL into the first half-chamber 7, air is vented from the latter through the vent opening 13.

In practice it has been verified that the invention achieves the intended purposes.

The invention as conceived is susceptible to modifications and variants, all of which are within the scope of the inventive concept.

Furthermore, all the details can be replaced with other technically equivalent elements.

In their practical embodiment, any other materials, as well as shapes and sizes, can be used according to requirements, without departing from the main field of protection of the following claims.

## Claims

1. Bioreactor (1) which comprises:
- a box-like body (4) having an internal culture chamber (5);
- a three-dimensional support (6) fitted in said internal chamber (5) for the support and growth of cells (2, 3) and which divides said internal chamber (5) into a first culture half-chamber (7) and a second culture half-chamber (8);
- a first opening (9) obtained in the box-like body (4) in correspondence with said first half-chamber (7);
- a second opening (10) obtained in the box-like body (4) in correspondence with said second half-chamber (8);
**characterized in that** at least one outward vent opening (13) is obtained in said box-like body (4) in correspondence with said first half-chamber (7).

2. The bioreactor as claimed in claim 1, wherein said at least one outward vent opening (13) is equipped with gas-permeable filtering means (14).

3. The bioreactor as claimed in claim 2, wherein said filtering means are hydrophobic and anti-contamination filtering means (14).

4. The bioreactor as claimed in claim 1, wherein said box-like-body (4) has a flattened shape and defines two parallel and opposite larger surfaces (4C, 4D), at least one of said larger surfaces being gas-permeable.

5. The bioreactor as claimed in claim 4, wherein said at least one of said larger surfaces (4C, 4D) consist of a hydrophobic and gas-permeable membrane.

6. The bioreactor as claimed in claim 1, wherein said at least one vent opening (13) is obtained in said first half-chamber (7) on the opposite side with respect to said first opening (9).

7. A cell culture method in a bioreactor (1) that has:
- a box-like body (4) that defines a culture chamber (5) inside it;
- a three-dimensional support (6) fitted in said culture chamber (5) and which divides it into a first culture half-chamber (7) and a second culture half-chamber (8);
- a first opening (9) obtained in said first culture half-chamber (7);
- a second opening (10) obtained in said second culture half-chamber (8), **characterized in that** it comprises:
- loading a second culture medium (TG) in a gel and/or semisolid state into said second culture half-chamber (8);
- loading a first culture medium (TL) in a liquid state into said first culture half-chamber (7);
- venting air from at least said first half-chamber (7) through a vent opening (13) obtained in said box-like body (4) in correspondence with said first culture half-chamber (7).
